(19) Europäisches Patentamt
European Patent Office
Office européen des brevets

(11) Veröffentlichungsnummer: **0 243 636 A1**

(12) # EUROPÄISCHE PATENTANMELDUNG

(21) Anmeldenummer: **87103585.3**

(22) Anmeldetag: **12.03.87**

(51) Int. Cl.³: **C 07 D 231/40**
C 07 D 231/38, C 07 D 401/04
A 01 N 43/56, A 01 N 43/40

(30) Priorität: **20.03.86 DE 3609423**

(43) Veröffentlichungstag der Anmeldung:
**04.11.87 Patentblatt 87/45**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Stetter, Jörg, Dr.**
**Gellertweg 4**
**D-5600 Wuppertal 1(DE)**

(72) Erfinder: **Schallner, Otto, Dr.**
**Noldweg 22**
**D-4019 Monheim(DE)**

(72) Erfinder: **Lindig, Markus, Dr.**
**Dahlienweg 16**
**D-4010 Hilden(DE)**

(72) Erfinder: **Jensen-Korte, Uta, Dr.**
**Geibelstrasse 9**
**D-4000 Düsseldorf(DE)**

(72) Erfinder: **Baasner, Bernd, Dr.**
**Hambergerstrasse 27d**
**D-5090 Leverkusen 3(DE)**

(72) Erfinder: **Becker, Benedikt, Dr.**
**Metzkausener Strasse 14**
**D-4020 Mettmann(DE)**

(72) Erfinder: **Homeyer, Bernhard, Dr.**
**Obere Strasse 28**
**D-5090 Leverkusen 3(DE)**

(72) Erfinder: **Behrenz Wolfgang, Dr.**
**Untergründemich 14**
**D-5063 Overath(DE)**

(72) Erfinder: **Stendel, Wilhelm, Dr.**
**In den Birken 55**
**D-5600 Wuppertal 1(DE)**

(54) **Schädlingsbekämpfungsmittel auf Basis von 1-Aryl-4-trifluormethyl-5-aminopyrazolen.**

(57) Es wurde gefunden, daß die teilweise bekannten 5-Aminopyrazole der allgemeinen Formel (I)

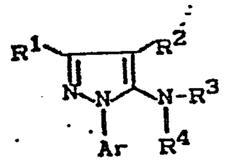

(I)

in welcher

$R^1$ für Wasserstoff, Alkyl oder Halogenalkyl steht,
$R^2$ für Trifluormethyl steht,
$R^3$ für Wasserstoff, Alkyl oder Alkenyl steht,
$R^4$ für Wasserstoff, Alkyl, Alkenyl, Alkinyl oder einen Rest

$-\underset{X}{\overset{\|}{C}}-R^5$ steht,

Ar für jeweils gegebenenfalls substituiertes Phenyl oder Pyridyl steht, wobei
X für Sauerstoff oder Schwefel steht, und
$R^5$ Alkyl oder Halogenalkyl bedeutet,

stark ausgeprägte insektizide, akarizide und nematizide Eigenschaften besitzen.

EP 0 243 636 A1

0243636

BAYER AKTIENGESELLSCHAFT 5090 Leverkusen, Bayerwerk
Konzernverwaltung RP
Patentabteilung Er/bo/c
IIa(+Ia)

# Schädlingsbekämpfungsmittel auf Basis von 1-Aryl-4-trifluormethyl-5-aminopyrazolen

Die Erfindung betrifft die Verwendung von teilweise bekannten 5-Aminopyrazolderivaten als Schädlingsbekämpfungsmittel, insbesondere als Insektizide und Akarizide.

Die insektizide Wirksamkeit von N,N-Dimethyl-O-pyrazolylcarbaminsäureestern ist bereits bekannt (siehe dazu DE-OS 28 39 270).

Die Wirkungshöhe bzw. die Wirkungsdauer dieser Verbindungen sind jedoch, insbesondere bei bestimmten Insekten oder bei niedrigen Anwendungskonzentrationen nicht immer völlig zufriedenstellend.

Es sind weiterhin bestimmte 5-Amino-1-aryl-pyrazole bekannt (vgl. z.B. Pharmaco. Ed. Sci. 26, 276-293 [1971] oder Mycopathologica 74, 7-14 [1981] bzw. C.A. 96: 1964 11j, sowie C.A. 95: 36257 q, DE-OS 34 02 308, publiziert am 01.08.1985).

Über eine Wirksamkeit dieser Verbindungsklasse gegen Insekten, Milben oder Nematoden ist bisher jedoch nichts bekannt.

Es wurde gefunden, daß die teilweise bekannten 5-Aminopyrazole der allgemeinen Formel (I)

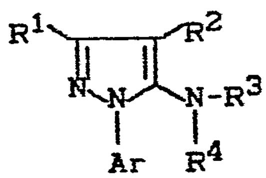

(I)

in welcher

$R^1$ für Wasserstoff, Alkyl oder Halogenalkyl steht,

$R^2$ für Trifluormethyl steht,

$R^3$ für Wasserstoff, Alkyl oder Alkenyl steht,

$R^4$ für Wasserstoff, Alkyl, Alkenyl, Alkinyl oder einen Rest 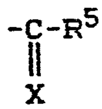 steht,

Ar für jeweils gegebenenfalls substituiertes Phenyl oder Pyridyl steht,

wobei X für Sauerstoff oder Schwefel steht,

und $R^5$ Alkyl oder Halogenalkyl bedeutet,

stark ausgeprägte insektizide und akarizide Eigenschaften besitzen.

Überraschenderweise zeigen die erfindungsgemäß zu verwendenden 5-Aminopyrazole der allgemeinen Formel (I) eine erheblich verbesserte insektizide, akarizide und nematizide Wirksamkeit als die aus dem Stand der Technik bekannten Pyrazolderivate

Die erfindungsgemäß zu verwendenden 5-Aminopyrazolderivate sind durch die Formel (I) allgemein definiert. Bevorzugt sind diejenigen Verbindungen der Formel (I), bei welchen

$R^1$ für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen oder für Wasserstoff steht,

$R^2$ für Trifluormethyl steht,

$R^3$ für Wasserstoff, $C_1$-$C_6$-Alkyl oder $C_2$-$C_6$-Alkenyl steht,

$R^4$ für Wasserstoff, $C_1$-$C_6$-Alkyl, $C_2$-$C_6$-Alkenyl, $C_2$-$C_6$-Alkinyl oder einen Rest $-\underset{\underset{O}{\|}}{C}-R^5$ steht,

wobei $R^5$ für $C_1$-$C_4$-Alkyl oder $C_1$-$C_4$-Halogenalkyl steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^6$

wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht.

Besonders bevorzugt sind diejenigen Verbindungen der Formel (I), bei welchen

$R^1$ für Wasserstoff, Methyl, Ethyl, n- oder i-Propyl oder Trifluormethyl steht,

$R^2$ für Trifluormethyl steht,

$R^3$ für Wasserstoff, Methyl, Ethyl, n- oder i-Propyl, n-, i-, s- oder t-Butyl, n- oder i-Pentyl, n- oder i-Hexyl, Vinyl, Propenyl-1, Propenyl-2, Butenyl-1, Butenyl-2, steht,

$R^4$ für Wasserstoff, Methyl, Ethyl, n- oder i-Propyl, n-, i-, s- oder t-Butyl, n- oder i-Pentyl, n- oder i-Hexyl, Propargyl, Butinyl-1, Butinyl-2 oder für einen der folgenden Reste steht:

$-\underset{\underset{O}{\|}}{C}-CH_3$ , $-\underset{\underset{O}{\|}}{C}-C_2H_5$ , $-\underset{\underset{O}{\|}}{C}-C_3H_7\text{-n}$ , $-\underset{\underset{O}{\|}}{C}-C_3H_7\text{-i}$ ,

$-\underset{\underset{O}{\|}}{C}-C_4H_9\text{-n}$ , $-\underset{\underset{O}{\|}}{C}-C_4H_9\text{-s}$ , $-\underset{\underset{O}{\|}}{C}-C_4H_9\text{-t}$ , $-\underset{\underset{O}{\|}}{C}-CF_3$

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen:
Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^6$, wobei

$R^6$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht

und

m für eine Zahl 0, 1 oder 2 steht.

Im einzelnen seien außer den bei den Herstellungsbeispielen genannten Verbindungen die folgenden 5-Aminopyrazole der allgemeinen Formel (I) genannt:

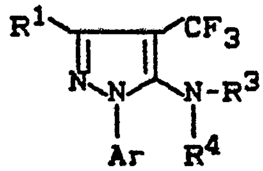

| $R^1$ | $R^3$ | $R^4$ | Ar |
|---|---|---|---|
| H | $CH_3$ | H | 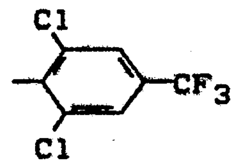 |
| H | $C_2H_5$ | H | " |
| H | $-CH_2-CH=CH_2$ | H | " |
| $CH_3$ | H | H | " |
| $CH_3$ | $CH_3$ | H | " |
| $CF_3$ | H | H | " |
| $CF_3$ | $CH_3$ | H | " |
| $CH_3$ | H | $COCH_3$ | " |
| H | H | H | 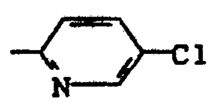 |
| H | $CH_3$ | H | " |
| H | $C_2H_5$ | H | " |
| $CH_3$ | H | H | " |
| $CH_3$ | $CH_3$ | H | " |

| $R^1$ | $R^3$ | $R^4$ | Ar |
|---|---|---|---|
| $CF_3$ | H | H | " |
| $CF_3$ | $CH_3$ | H | " |
| H | H | $COCH_3$ | " |
| $CH_3$ | H | $COCH_3$ | " |
| $C_2H_5$ | $CH_3$ | $COCH_3$ | " |
| H | H | H | 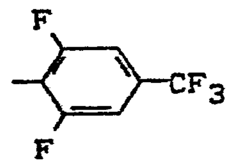 |
| H | $CH_3$ | H | " |
| H | $C_2H_5$ | H | " |
| H | $-CH_2-CH=CH_2$ | H | " |
| $CH_3$ | H | H | " |
| $CH_3$ | $CH_3$ | H | " |
| $CF_3$ | H | H | " |
| $CF_3$ | $CH_3$ | H | " |
| H | H | $COCH_3$ | " |
| $CH_3$ | H | $COCH_3$ | " |
| $C_2H_5$ | $CH_3$ | H | " |
| H | H | H | 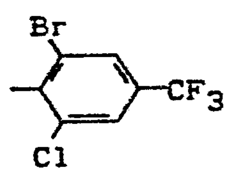 |

| $R^1$ | $R^3$ | $R^4$ | Ar |
|---|---|---|---|
| H | $CH_3$ | H | " |
| H | $C_2H_5$ | H | " |
| H | $-CH_2-CH=CH_2$ | H | " |
| $CH_3$ | H | H | " |
| $CH_3$ | $CH_3$ | H | " |
| $CF_3$ | H | H | " |
| $CF_3$ | $CH_3$ | H | " |
| H | H | $COCH_3$ | " |
| $CH_3$ | H | $COCH_3$ | " |
| $C_2H_5$ | $CH_3$ | H | 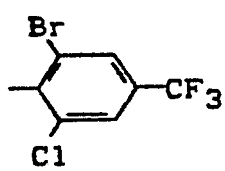 |
| H | H | H | 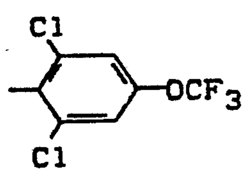 |
| H | $CH_3$ | H | " |
| H | $C_2H_5$ | H | " |
| H | $-CH_2-CH=CH_2$ | H | " |
| $CH_3$ | H | H | " |
| $CH_3$ | $CH_3$ | H | " |
| $CF_3$ | H | H | " |
| $CF_3$ | $CH_3$ | H | " |

| $R^1$ | $R^3$ | $R^4$ | Ar |
|---|---|---|---|
| H | H | $COCH_3$ | " |
| $CH_3$ | H | $COCH_3$ | " |
| H | H | $-CH_2-C \equiv CH$ | " |
| $C_2H_5$ | $CH_3$ | H | " |
| H | H | H | 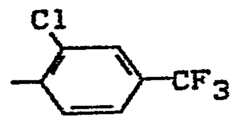 |
| H | $CH_3$ | H | " |
| H | $C_2H_5$ | H | " |
| H | $-CH_2-CH=CH_2$ | H | " |
| $CH_3$ | H | H | " |
| $CH_3$ | $CH_3$ | H | 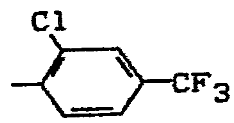 |
| $CF_3$ | H | H | " |
| $CF_3$ | $CH_3$ | H | " |
| H | H | $COCH_3$ | " |
| $CH_3$ | H | $COCH_3$ | " |
| $C_2H_5$ | H | H | " |
| $C_2H_5$ | $CH_3$ | H | " |
| H | $CH_3$ | $CH_3$ | " |

Die erfindungsgemäß zu verwendenden 5-Aminopyrazole der Formel (I) sind teilweise bekannt (vgl. DE-OS 34 02 308) und können in Analogie zu den dort beschriebenen Herstellungsverfahren erhalten werden.

Noch nicht bekannt sind substituierte 5-Amino-1-phenylpyrazole der Formel (Ia)

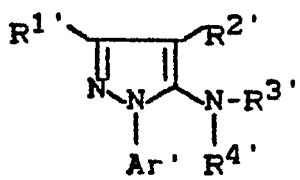

(Ia)

in welcher

$R^{1'}$ für Wasserstoff, Alkyl oder Halogenalkyl steht,

$R^{2'}$ für Trifluormethyl steht,

$R^{3'}$ für Wasserstoff, Alkyl oder Alkenyl steht,

$R^{4'}$ für Wasserstoff, Alkyl, Alkenyl, Alkinyl oder einen Rest

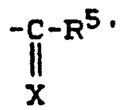 steht

Ar' für jeweils gegebenenfalls substituiertes Phenyl oder Pyridyl steht,

wobei

X für Sauerstoff oder Schwefel steht und

$R^{5'}$ für Alkyl oder Halogenalkyl steht,

wobei, falls $R^{1'}$ für Wasserstoff steht, die Reste $R^{3'}$, $R^{4'}$ und Ar' nicht die folgenden Bedeutungskombinationen besitzen dürfen:

$R^{3'}$ = Wasserstoff, $R^{4'}$ = Wasserstoff und Ar' = 2,4,6-Trichlorphenyl oder 2,4,6-Trinitrophenyl;

$R^{3'}$ = Wasserstoff, $R^{4'}$ = $COCH_3$ und Ar' = 2,6-Dichlor-4-trifluormethylphenyl oder 2,6-Dichlor-4-trifluormethyloxyphenyl;

$R^{3'}$ = Wasserstoff, $R^{4'}$ = $COC_2H_5$ und Ar' = 2,6-Dichlor-4-trifluormethylthiophenyl oder 2,3,6-Trifluor-4-trifluormethylthiophenyl;

$R^{3'}$ = Methyl, $R^{4'}$ = $COCH_3$ und Ar' = 2-Chlor-4-trifluormethylphenyl.

Man erhält

(a) die 1-Aryl-5-amino-4-trifluormethylpyrazole der Formel (Ia),

wenn man

1-Aryl-5-amino-4-carboxypyrazole der Formel (II)

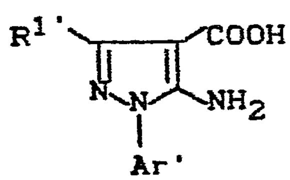

(II)

in welcher

$R^{1'}$ und Ar' die oben angegebene Bedeutung besitzen,

mit Schwefeltetrafluorid in Fluorwasserstoffsäure unter erhöhtem Druck umsetzt,

und die entstehenden Verbindungen der Formel

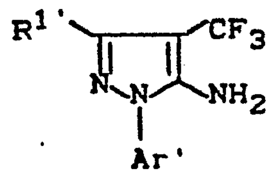

(Ib)

gegebenenfalls alkyliert, alkenyliert, alkinyliert oder acyliert,

oder man erhält

(b) die 1-Aryl-5-amino-4-trifluormethylpyrazole der Formel

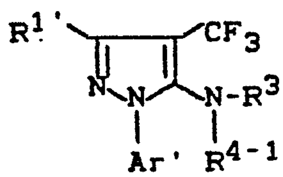

(Ic)

in welcher

$R^{4-1}$ für Alkyl, Alkenyl, Alkinyl oder für einen Rest

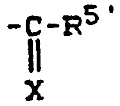

steht und

$R^{1'}$, $R^{3'}$, $R^{5'}$, Ar' und X die oben angegebene Bedeutung haben,

wenn man

1-Aryl-5-amino-4-trifluormethylpyrazole der Formel

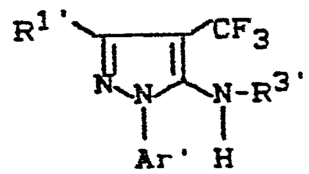

(Id)

in welcher

$R^{1'}$, $R^{3'}$ und Ar' die oben angegebene Bedeutung haben,

mit Verbindungen der Formel

$R^{4-1}$-A (III)

in welcher

$R^{4-1}$ für Alkyl, Alkenyl, Alkinyl oder für einen Rest 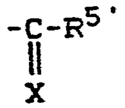 steht, wobei

$R^{5'}$ und X die oben angegebene Bedeutung haben und

A für eine elektronenziehende Abgangsgruppe steht,

gegebenenfalls in Gegenwart eines Verdünnungsmittels und gegebenenfalls in Gegenwart eines Säurebindemittels umsetzt,

oder man erhält

(c) die 1-Aryl-5-amino-4-trifluormethylpyrazole der Formel

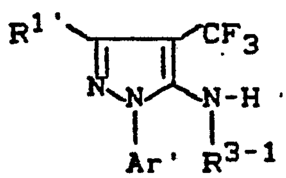

(Ie)

in welcher

$R^{1'}$ und Ar' die oben angegebene Bedeutung haben und

$R^{3-1}$ für Alkyl oder Alkenyl steht,

indem man

5-(N-Acylamino)-4-trifluormethyl-1-aryl-pyrazole der Formel

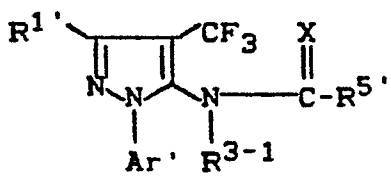

(If)

in welcher

$R^{1'}$, $R^{5'}$, $R^{3-1}$, X und Ar' die oben angegebene Bedeutung haben,

mit Säuren oder Basen, gegebenenfalls in Gegenwart eines Verdünnungsmittels deacyliert,

oder man erhält

(d) die 1-Aryl-5-amino-trifluormethylpyrazole der Formel

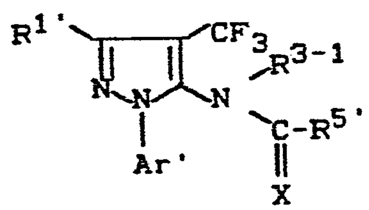

(Ig)

in welcher

$R^{1'}$, $R^{3-1}$, $R^{5'}$, X und Ar' die oben angegebene Bedeutung haben,

indem man

1-Aryl-5-amino-trifluormethylpyrazole der Formel

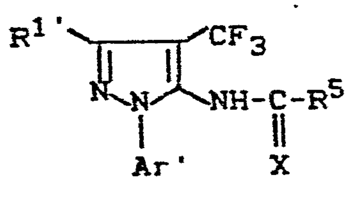

(Ih)

mit Verbindungen der Formel $R^{3-1}$-A

in welcher

$R^{3-1}$ die oben angegebene Bedeutung besitzt und A für eine Abgangsgruppe steht, umsetzt.

Verwendet man bei der Herstellungsvariante (a) 1-(2,6-Dichlor-4-trifluormethylphenyl)-4-carboxy-5-aminopyrazol und Schwefeltetrafluorid als Ausgangsstoffe, so läßt sich der Reaktionsablauf durch das folgende Formelschema darstellen:

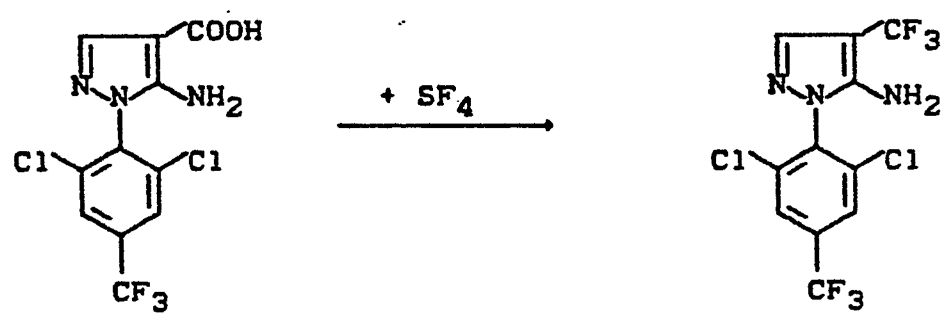


Verwendet man bei der Herstellungsvariante (b) 1-(2,6-Dichlor-4-trifluormethyl-phenyl)-3-methyl-4-trifluormethyl-5-aminopyrazol und Propionylchlorid als Ausgangsstoffe, so läßt sich der Reaktionsablauf durch das folgende Formelschema darstellen:

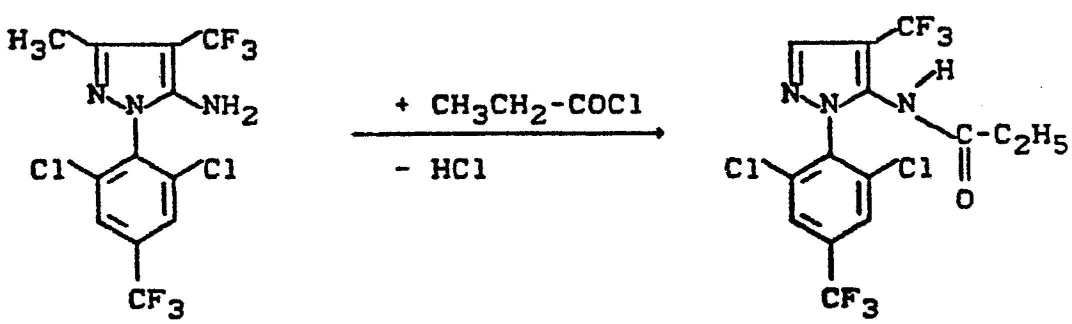

Verwendet man bei der Herstellungsvariante c) 1-(2,6-Dichlor-4-trifluormethyl-phenyl)-4-trifluormethyl-5-(ethyl-acetylamino)-pyrazol als Ausgangsprodukt und NaOH als Base, so kann der Reaktionsablauf wie folgt dargestellt werden:

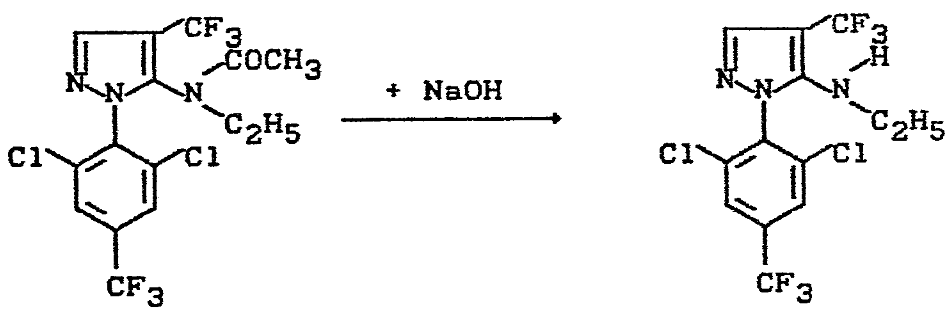


Verwendet man bei der Herstellungsvariante (d) 1-(2,6-Dichlor-4-trifluormethylphenyl)-4-trifluormethyl-5-acetylamino-pyrazol und Ethylchlorid als Ausgangsprodukte, so kann der Reaktionsablauf wie folgt dargestellt werden:

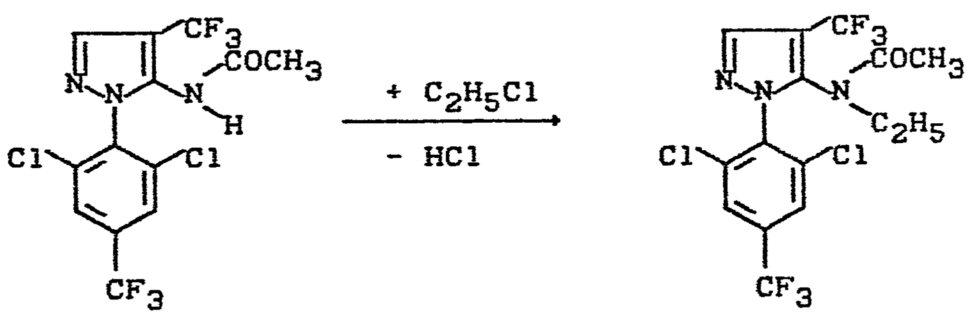


Die zur Durchführung des erfindungsgemäßen Verfahrens (a) als Ausgangsstoffe benötigten 1-Aryl-5-amino-4-carboxypyrazole sind durch die Formel (II) allgemein definiert.

In dieser Formel stehen $R^1$ und Ar vorzugsweise für diejenigen Reste, die bereits im Zusammenhang mit der Beschreibung der Stoffe der Formel (I) als bevorzugt für diese Substituenten genannt wurden.

Die 1-Aryl-5-amino-4-carboxypyrazole der Formel (II)

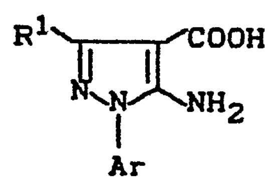

(II)

sind teilweise bekannt (vgl. z.B. J. Org. Chem. 36, 2972 - 2974 [1971] oder J. Heterocyclic Chemistry 7, 345 - 349 [1970], C.A. 62: 13137 c).

Man erhält sie beispielsweise, wenn man Arylhydrazine der Formel

$$Ar\text{-}NH\text{-}NH_2 \qquad (IV)$$

in welcher

Ar die oben angegebene Bedeutung besitzt,

mit Acrylnitrilderivaten der Formel

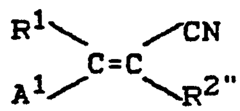

(V)

in welcher

$R^1$ für Wasserstoff, Alkyl oder Halogenalkyl steht,

$R^{2"}$ für Alkoxycarbonyl steht und

$A^1$ für Halogen, Hydroxy, Alkoxy, Amino oder Dialkylamino steht,

entweder zunächst in einer 1. Stufe, gegebenenfalls in Gegenwart eines Verdünnungsmittels wie beispielsweise Ethanol oder Eisessig und gegebenenfalls in Gegenwart eines Reaktionshilfsmittels wie beispielsweise Natriumacetat bei Temperaturen zwischen -20° C und +20° C umsetzt zu den Arylhydrazinderivaten der Formel (VI),

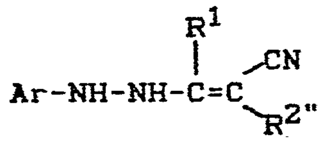

(VI)

in welcher

Ar, $R^1$ und $R^{2"}$ die oben angegebene Bedeutung haben,

und diese in einer 2. Stufe, gegebenenfalls in Gegenwart eines Verdünnungsmittels wie beispielsweise Ethylenglykolmonoethylether bei Temperaturen zwischen +50° C und +150° C cyclisiert, oder direkt in einem Reaktionsschritt ohne Isolierung der Zwischenstufe der Formel (VI), gegebenenfalls in Gegenwart eines Verdünnungsmittels wie beispielsweise Ethylenglykolmonoethylether bei Temperaturen zwischen +50° C und +150° C direkt cyclisiert zu den 5-Aminopyrazolen der Formel (VII)

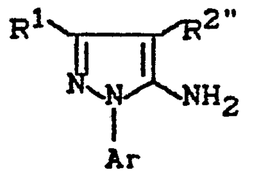 (VII)

in welcher

$R^1$, $R^{2"}$ und Ar die oben angegebene Bedeutung haben,

und die erhaltenen 5-Amino-pyrazol-Derivate der Formel (VI) in allgemein üblicher Art und Weise, gegebenenfalls in Gegenwart eines Verdünnungsmittels, wie beispielsweise Ethanol oder Isopropanol bei Temperaturen zwischen +20° C und 140° C zu 4-Carboxy-5-amino-pyrazolen der Formel (II)

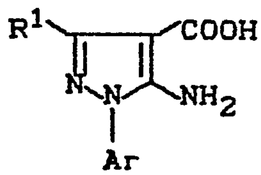 (II)

verseift.

Die Arylhydrazine der Formel

$$Ar-NH-NH_2 \qquad (IV)$$

sind bekannt (vgl. z.B. US-PS 4 127 575; US-PS 3 609 158; DE-OS 2 558 399; J. Chem. Soc. C, 1971, 167 - 174) oder

lassen sich nach prinzipiell bekannten Verfahren in einfacher analoger Weise herstellen (vgl. z.B. Houben-Weyl, "Methoden der organischen Chemie", Band X, 2 S. 203, Thieme Verlag Stuttgart 1967).

Die Acrylnitrilderivate der Formel (V)

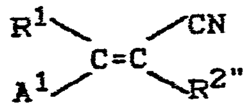

(V)

sind allgemein bekannte Verbindungen der organischen Chemie. Die Verbindungen der Struktur (Va)

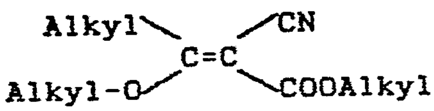

(Va)

wobei Alkyl für einen Alkylrest steht (vorzugsweise $C_1$-$C_6$-Alkyl), sind z.B. bekannt aus

Beilstein E II 3, S. 301,
F. Arndt et al., Ann. <u>521</u>, 95 (1935),
T. Hayashi et al., J.O.C. <u>30</u>, 695 (1965),
J. Hori et al., Sci. Papers Inst. Phys. Chem. Res. <u>56</u>, 216 (1962).

Die Acrylnitrilderivate der Formel (V)

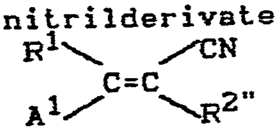

(V)

wobei

$R^1$ für Niederalkyl steht,

$A^1$ für Niederalkoxy steht und

$R^{2"}$ für Alkoxycarbonyl steht,

sind beispielsweise durch Umsetzung von Cyanessigestern mit Orthoestern erhältlich, wie z.B. durch folgende Umsetzungen:

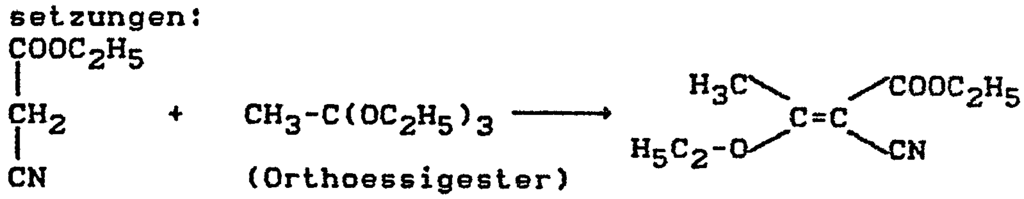

(Orthoessigester)

oder

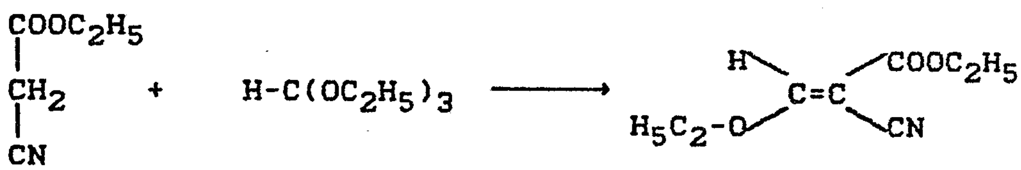

(Orthoameisensäureester)

Die zur Durchführung der Verfahrensvariante (b) als Ausgangsstoffe benötigten 1-Aryl-5-amino-4-trifluormethyl-pyrazole sind durch die Formel (Ic) allgemein definiert.

In dieser Formel stehen $R^1$, $R^3$ und Ar vorzugsweise für diejenigen Reste, die bereits im Zusammenhang mit der Beschreibung der Stoffe der Formel (I) als bevorzugt für diese Substituenten genannt wurden.

Die Verbindungen der Formel (Ic) sind erfindungsgemäße Verbindungen, man erhält sie beispielsweise mit Hilfe der Verfahrensvariante (a) oder (d).

Die zur Durchführung des Verfahrens (b) weiterhin als Ausgangsstoffe benötigten Verbindungen sind durch die Formel (III) allgemein definiert. In dieser Formel steht A vorzugsweise für Chlor, Brom, Iod, p-Toluolsulfonyloxy, Alkoxysulfonyloxy oder Acyloxy, $R^{4-1}$ steht vorzugsweise für Alkyl mit 1 bis 8 Kohlenstoffatomen, Alkenyl oder Alkinyl mit jeweils 2 bis 8 Kohlenstoffatomen oder für den Rest $-\underset{\underset{X}{\|}}{C}-R^5$

wobei X vorzugsweise für ein Sauerstoffatom steht und $R^5$ vorzugsweise für Alkyl mit 1 bis 8 Kohlenstoffatomen steht.

Die zur Durchführung der Verfahrensvariante (c) als Ausgangsstoffe benötigten 5-(N-Acylamino)-4-trifluormethyl-1-arylpyrazole sind durch die Formel (If) allgemein definiert.

In dieser Formel stehen $R^{1'}$, $R^{5'}$, X und Ar' vorzugsweise für diejenigen Reste, die bereits im Zusammenhang mit der Definition von Verbindungen der Formel (I) angegeben worden sind. $R^{3-1}$ steht vorzugsweise für Alkyl mit 1 bis 8 Kohlenstoffatomen oder Alkenyl mit 2 bis 8 Kohlenstoffatomen.

Die Verbindungen der Formel (If) sind erfindungsgemäße Verbindungen und erhältlich mit Hilfe der Verfahrensvariante (b) oder (d).

Bei der Verfahrensvariante (a) setzt man Verbindungen der Formel (II) mit Fluorwasserstoffsäure und Schwefeltetrafluorid vorzugsweise im Autoklaven gegebenenfalls unter

Inertgasatmosphäre (insbesondere Stickstoffatmosphäre), d.h. unter erhöhtem Druck einige Stunden bei Temperaturen von 60 - 200°C, insbesondere von 80 - 150°C, um, entspannt, destilliert noch verbleibende Fluorwasserstoffsäure ab und arbeitet den Rückstand in an sich bekannter Weise auf (beispielsweise gießen auf Eiswasser, Zutropfen von Alkalilauge, Waschen mit organischem Lösungsmittel und chromatographische Reinigung des Endprodukts).

Hierbei setzt man pro Mol Verbindung der Formel (II) vorzugsweise 10 bis 100 Mol Fluorwasserstoffsäure und 1,5 bis 10 Mol, insbesondere 2 bis 5 Mol, Schwefeltetrafluorid um.

Bei den Verfahrensvarianten (b) und (d) kommen als Verdünnungsmittel inerte organische Lösungsmittel in Frage. Vorzugsweise verwendet man folgende Lösungsmittel:

aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether wie Diethylether, Diisopropylether, Dioxan, Tetrahydrofuran oder Ethylenglykoldiethylether oder -dimethylether, Ketone wie Aceton, Butanon, Methylisopropylketon oder Methylisobutylketon, Ester wie Essigsäureethylester, Nitrile, wie Acetonitril oder Propionitril, Amide wie Dimethylformamid, Dimethylacetamid, N-Methylpyrrolidon oder Hexamethylphosphorsäuretriamid. Die Verfahrensvarianten (b) und (d)

können aber auch in Alkohol oder im wäßrigen System, beispielsweise zweiphasig unter Zusatz von Phasentransferkatalysatoren durchgeführt werden.

Es ist jedoch auch möglich, die als Reaktionspartner verwendeten Verbindungen der Formel (III) in entsprechendem Überschuß als Verdünnungsmittel einzusetzen.

Als Säurebindemittel zur Durchführung der Herstellungsverfahren (b) und (d) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung der Herstellungsverfahren (b) und (d) in einem größeren Bereich variiert werden. Im allgemeinen arbeitet man zwischen -20° C und +150° C.

Bei der Verfahrensvariante (c) kommen als Verdünnungsmittel inerte organische oder anorganische Lösungsmittel in Frage, insbesondere kommen Alkohole wie Methanol oder Ethanol oder deren Gemische mit Wasser in Frage.

Das Verfahren (c) wird entweder in Gegenwart einer starken Säure, wie beispielsweise Salzsäure, Trifluoressigsäure oder Bromwasserstoffsäure, in Eisessig oder in Gegenwart

einer Base durchgeführt. Als Basen sind wäßrige Lösungen von Natriumhydroxid oder Kaliumhydroxid bevorzugt.

Die Reaktionstemperaturen können bei der Durchführung des erfindungsgemäßen Verfahrens (c) in einem größeren Bereich variiert werden. Im allgemeinen arbeitet man bei Temperaturen zwischen -20° C und +150° C, vorzugsweise bei Temperaturen zwischen 0° C und +120° C.

Zur Durchführung des Verfahrens (c) setzt man pro Mol 5-(N-Acylamino)-4-trifluormethyl-1-aryl-pyrazol der Formel (If) im allgemeinen 1 bis 30 Mol, vorzugsweise 1 bis 15 Mol, an Säure oder Base ein. Die Reaktionsdurchführung, Aufarbeitung und Isolierung der Reaktionsprodukte der Formel (Ie) erfolgt im allgemein üblicher Art und Weise.

Die Wirkstoffe eignen sich bei guter Pflanzenverträglichkeit und günstiger Warmblütertoxizität zur Bekämpfung von tierischen Schädlingen, insbesondere Insekten und Spinnentieren die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.

Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

0243636

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäß verwendbaren Wirkstoffe der Formel (I) zeichnen sich durch eine hohe insektizide und akarizide Wirksamkeit aus. Sie lassen sich insbesondere gegen pflanzenschädigende Insekten, wie beispielsweise gegen die Raupen der Kohlschabe (Plutella maculipennis) oder gegen die Larven der Meerrettichblattkäfer (Phaedon cochleariae), sowie gegen pflanzenschädigende Milben, wie beispielsweise gegen die gemeine Spinnmilbe (Tetranychus urticae) einsetzen. Sie eignen sich daneben hervorragend

zur Bekämpfung von Bodeninsekten und lassen sich beispielsweise zur Bekämpfung von Phorbia antiqua-Maden Myzus persicae und Diabrotica balteata einsetzen. Auch eine nennenswerte wurzelsystemische Wirkung, beispielsweise gegen Phaedon cochleariae-Larven ist hervorzuheben.

Außerdem besitzen die erfindungsgemäß verwendbaren Wirkstoffe der Formel (I) eine hohe Wirkung gegen Hygiene und Vorratsschädlinge und lassen sich beispielsweise zur Bekämpfung der orientalischen Schabe (Blatta orientalis), der deutschen Schabe (Blatella germanica) oder zur Bekämpfung des gemeinen Kornkäfers (Sitophilus granarius) einsetzen. Darüber hinaus lassen sich die erfindungsgemäßen Wirkstoffe mit besonders gutem Erfolg zur Bekämpfung von parasitisch lebenden Warmblüterschädlingen, (sowohl Ekto- als auch Endoparasiten), wie beispielsweise gegen die Larven der Goldfliege (Lucilia cuprina), gegen Rinderzecken (Boophilus microplus), gegen Räudemilben (Psoroptes ovis), gegen Stechfliegen (Stomoxys calcitrans) oder gegen die Weideviehfliege (Musca autumnalis) einsetzen.

Daneben besitzen die erfindungsgemäß verwendbaren Wirkstoffe der Formel (I) auch eine gute wachstumsregulierende Wirkung bei Pflanzen.

In entsprechenden Aufwandmengen zeigen die erfindungsgemäß verwendbaren Wirkstoffe der Formel (I) darüber hinaus eine herbizide Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterialantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäß verwendbaren Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variiert werden. Die Wirkstoffkonzentration der Anwendungsformen kann von 0,0000001 bis zu 95 Gew.-% Wirkstoff, vorzugsweise zwischen 0,0001 und 1 Gew.-% liegen.

0243636

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesen Gebieten in bekannter Weise, wie durch äußerliche Anwendung in Form beispielsweise des Tauchens (Dippen, Sprühens (Sprayen), Aufgießens (pour-on and spot-on) und des Einpuderns.

Die Herstellung und die Verwendung der Wirkstoffe geht aus den nachfolgenden Beispielen hervor.

## Herstellungsbeispiele:

### Beispiel 1

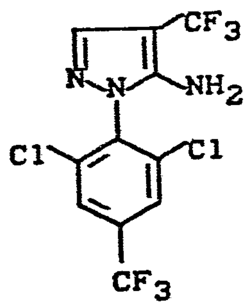

30,8 g (0,1 Mol) 5-Amino-1-(2,6-dichlor-4-trifluormethyl-phenyl)-4-carboxypyrazol, 100 ml wasserfreie Fluorwasserstoffsäure und 40 g Schwefeltetrafluorid werden in einem Stahlrührautoklaven unter Stickstoffatmosphäre 8 Stunden bei 120° C gerührt. Anschließend wird bei Raumtemperatur entspannt, noch verbliebene Fluorwasserstoffsäure abdestilliert und der Rückstand auf 500 ml Eiswasser gegossen. Durch Zutropfen von konzentrierter Natronlauge wird dieses Gemisch alkalisch gestellt. Der entstehende Feststoff wird abgesaugt und mit Diethylether nachgewaschen. Das Filtrat wird zweimal mit Diethylether extrahiert. Nach dem Trocknen über wasserfreiem Magnesiumsulfat wird das Lösungsmittel entfernt und der verbleibende feste Rückstand über eine kurze Kieselgelsäule chromatographisch filtriert (Laufmittel: Methylenchlorid). Nach Entfernung des Lösungsmittels bleibt das reine Produkt zurück. Man erhält 26,6 g (73 % der Theorie) an 5-Amino-1-(2,6-dichlor-4-trifluormethyl-phenyl)-4-trifluormethylpyrazol vom Schmelzpunkt 106° C.

Nach dem gleichen Verfahren werden die in der nachfolgenden Tabelle aufgeführten Beispiele dargestellt, wobei in den Beispielen 2 und 3 als Laufmittel bei der kurzen Kieselgelsäule ein Gemisch Methylenchlorid/Petrolether 4:1 eingesetzt wird, bei den anderen Beispielen reines Methylenchlorid als Laufmittel.

Allgemeine Formel:

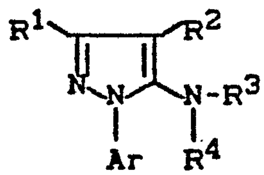

| Bsp. | $R^1$ | $R^2$ | $R^3$ | $R^4$ | Ar | Fp[°C] |
|---|---|---|---|---|---|---|
| 2 | H | $CF_3$ | H | H | 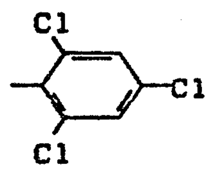 | 98-99 |
| 3 | H | $CF_3$ | H | H | 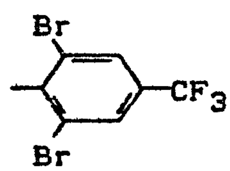 | 148-150 |
| 4 | $CH_3$ | $CF_3$ | H | H | 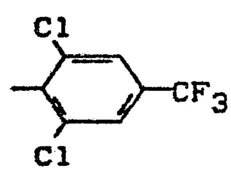 | 131-133 |
| 5 | $C_2H_5$ | $CF_3$ | H | H | 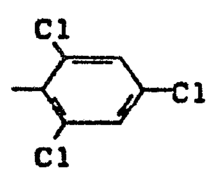 | 107-109 |

| Bsp. | $R^1$ | $R^2$ | $R^3$ | $R^4$ | Ar | Fp[°C] |
|---|---|---|---|---|---|---|
| 6 | H | $CF_3$ | H | H | 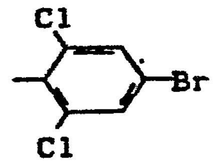 | 98 |
| 7 | H | $CF_3$ | H | H | 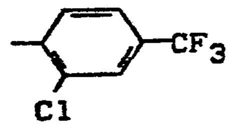 | 96-97 |
| 8 | H | $CF_3$ | H | H | 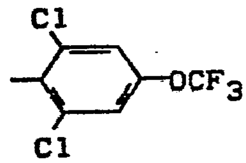 | |
| 9 | H | $CF_3$ | H | H | 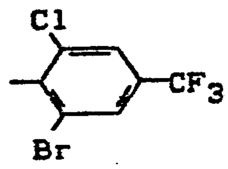 | |
| 10 | H | $CF_3$ | $CH_3$ | H | 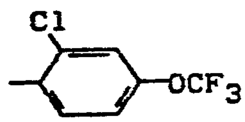 | |
| 11 | H | $CF_3$ | $CH_3$ | $CH_3$ | 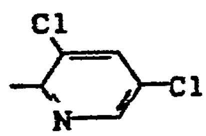 | |
| 12 | $CH_3$ | $CF_3$ | H | H | 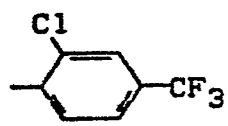 | |
| 13 | $CH_3$ | $CF_3$ | $C_2H_5$ | H | 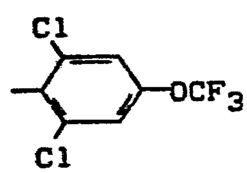 | |
| 14 | $CH_3$ | $CF_3$ | $C_2H_5$ | $C_2H_5$ | 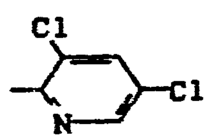 | |

Beispiel 15

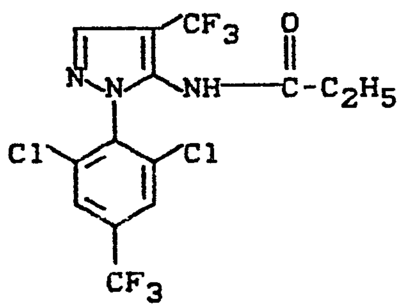

Eine Mischung aus 11 g (0,03 Mol) 5-Amino-1-(2,6-dichlor-4-trifluormethyl-phenyl)-4-trifluormethyl-pyrazol, 5 g Propionsäureanhydrid und 0,5 ml konzentrierter Schwefelsäure in 50 ml Acetonitril wird 8 Stunden bei 60°C gerührt. Nach dem Abkühlen wird der Ansatz auf Wasser gegossen, mit Methylenchlorid extrahiert, die organische Phase über Natriumsulfat getrocknet und anschließend im Vakuum eingeengt. Der Rückstand wird aus Ligroin umkristallisiert. Ausbeute: 9,3 g 5-Propionylamino-1-(2,6-dichlor-4-trifluormethyl phenyl)-4-trifluormethyl-pyrazol vom Schmelzpunkt 136 - 140°C.

Auf analogem Wege werden die im folgenden aufgeführten Beispiele 16-23 hergestellt.

Allgemeine Formel:

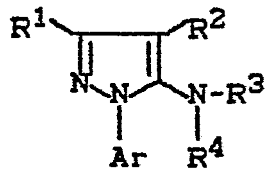

| Bsp. | $R^1$ | $R^2$ | $R^3$ | $R^4$ | Ar | Fp[°C] |
|---|---|---|---|---|---|---|
| 16 | H | $CF_3$ | H | $COC_2H_5$ | 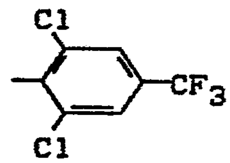 | 136-140 |
| 17 | H | $CF_3$ | H | $COCH_3$ | " | 126-130 |
| 18 | H | $CF_3$ | H | $COCF_3$ | " | 130 |
| 19 | H | $CF_3$ | H | $COCHCl_2$ | " | 135-138 |
| 20 | H | $CF_3$ | H | $COCH_2Cl$ | " | 128-130 |
| 21 | H | $CF_3$ | H | $COC_2H_5$ | 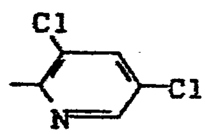 | |
| 22 | H | $CF_3$ | H | $COCH_3$ | " | |
| 23 | H | $CF_3$ | H | $COCF_3$ | " | |
| 24 | H | $CF_3$ | H | $COCH_3$ | 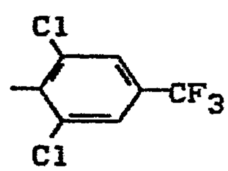 | 120-124 |
| 25 | H | $CF_3$ | H | $COC_2H_5$ | 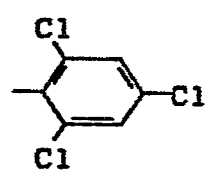 | 133 |

Herstellung von Ausgangsverbindungen:

Beispiel A)

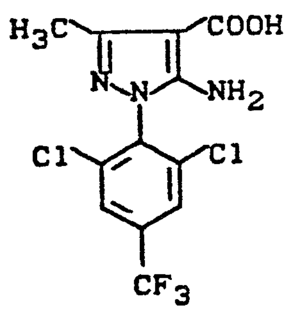

35,4 g (0,1 Mol) 5-Amino-1-(2,6-dichlor-4-trifluormethyl-phenyl)-4-carbethoxy-pyrazol werden in einer Lösung aus 8 g (0,2 Mol) Natriumhydroxid, 24 ml Wasser und 100 ml Ethanol 24 Stunden unter Rückfluß erhitzt. Anschließend wird mit 450 ml Wasser versetzt und zweimal mit 70 ml Diethylether gewaschen. Von der wäßrigen Phase wird der Alkohol abdestilliert und dann unter Eiskühlung mit 20 ml konzentrierter Salzsäure versetzt. Die Suspension wird auf 5° C abgekühlt, der Niederschlag abgsaugt, mit Wasser neutral gewaschen und getrocknet. Man erhält 26,2 g (74 % der Theorie) der Verbindung 5-Amino-1-(2,6-dichlor-4-trifluormethyl-phenyl)-4-carboxy-pyrazol vom Schmelzpunkt 158° C (Zersetzung).

In entsprechender Weise werden hergestellt:

Allgemeine Formel:

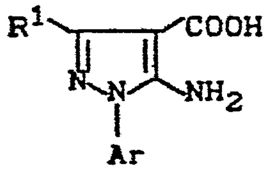

| $R^1$ | Ar | Fp[° C] |
|---|---|---|
| H | 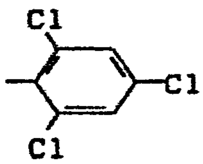 | 186 |
| H | 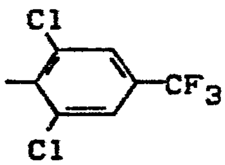 | 188-190 (Zers.) |
| H | 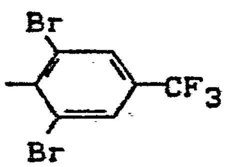 | 220 |
| $CH_3$ | 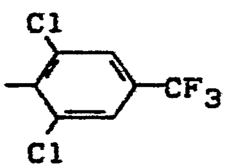 | 158 (Zers.) |
| $C_2H_5$ | 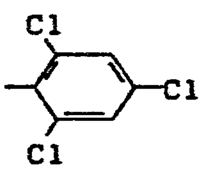 | 158 (Zers.) |
| H | 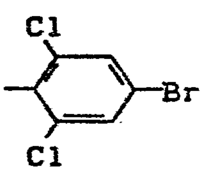 | 163 |

| $R^1$ | Ar | Fp[° C] |
|---|---|---|
| H | 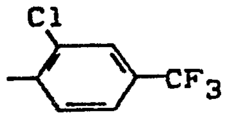 | 180 (Zers.) |
| H | 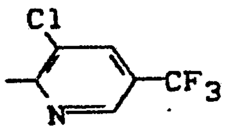 | |

Beispiel B)

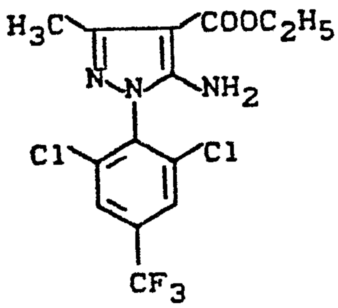

245 g (1 Mol) 2,6-Dichlor-4-trifluormethyl-phenylhydrazin und 183 g (1 Mol) Methyl-ethoxymethylen-malonsäuremonoethylesternitril werden in 2,5 l Ethanol gelöst und 10 Stunden bei Rückflußtemperatur gerührt. Anschließend wird das Lösungsmittel im Rotationsverdampfer entfernt und der Rückstand in Methylenchlorid aufgenommen. Dann wird mit Wasser gewaschen, in Diethylether über Magnesiumsulfat getrocknet, filtriert und eingeengt. Der Rückstand wird am Hochvakuum bei 90° C andestilliert. Es verbleiben 290 g (75 % der Theorie) 5-Amino-1-(2,6-dichlor-4-trifluormethyl-phenyl)-3-methyl-4-carbethoxy-pyrazol vom Schmelzpunkt 104 - 108° C.

In entsprechender Weise werden gemäß folgendem Schema hergestellt:

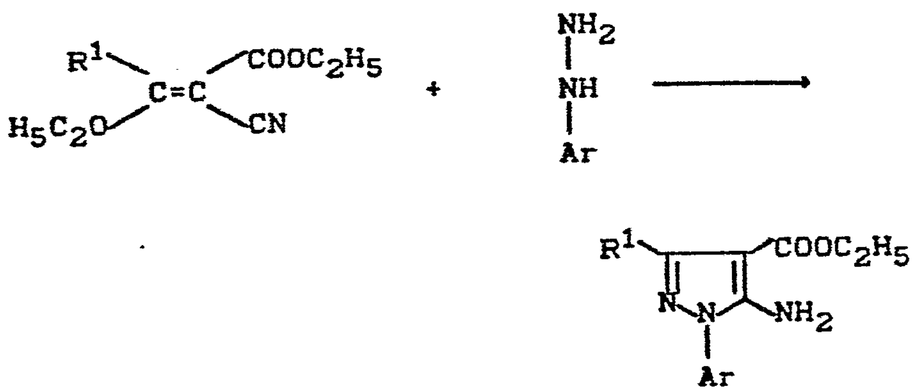

| $R^1$ | Ar | Fp[°C] |
|---|---|---|
| H | 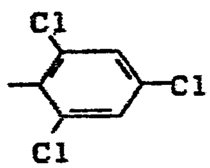 | 107 |
| H | 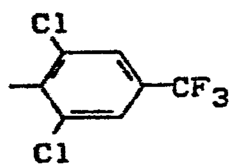 | 120-124 |
| H | 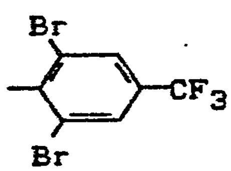 | 170 |
| $CH_3$ | 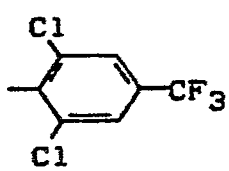 | 104-108 |
| $C_2H_5$ | 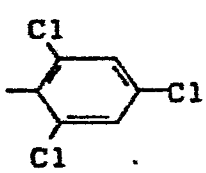 | 38-40 ($Kp_{0.7\ mm}$: 217°C) |

| $R^1$ | Ar | Fp[°C] |
|---|---|---|
| H | 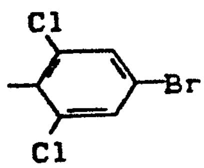 | 112-117 |
| H | 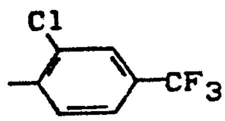 | 112-114 |

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 4, 7, 15, 17.

Beispiel B

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 4, 15, 17.

Beispiel C

Grenzkonzentrations-Test / Bodeninsekten

Testinsekt: Phorbia antiqua-Maden im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 3, 4, 15.

Beispiel D

Grenzkonzentrationstest / Bodeninsekten

Testinsekt: Diabrotica balteata - Larven im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Die Wirkstoffzubereitung wird innig mit dem Boden vermischt. Dabei spielt die Konzentration des Wirkstoffes in der Zubereitung praktisch keine Rolle, entsprechend ist allein die Wirkstoffgewichtsmenge pro Volumeneinheit Boden, welche in ppm (mg/l) angegeben wird. Man füllt den Boden in 0,5 l Töpfe und läßt diese bei $20^0$C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie in der unbehandelten Kontrolle.

Bei diesem Test zeigen z.B. die folgenden Verbindungen der Herstellungsbeispiele überlegene Wirkung gegenüber dem Stand der Technik: 4, 15.

## Beispiel E

Grenzkonzentrations-Test / Wurzelsystemische Wirkung

Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 15.

Beispiel F

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 15, 17.

Beispiel G

$LD_{100}$-Test

Testtiere: Sitophilus granarius
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 1, 4, 6, 15, 17, 18, 19.

Beispiel H

$LD_{100}$-Test

Testtiere: Blatella germanica
Zahl der Testtiere: 20
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirksamkeit gegenüber dem Stand der Technik: 1, 4, 15, 18, 19.

Beispiel J

$LT_{100}$-Test für Dipteren

Testtiere: Musca domestica
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirksamkeit gegenüber dem Stand der Technik: 1, 4, 17, 19.

0243636

Beispiel K

Test mit Boophilus microplus resistent/OP-resistenter Biarra-Stamm

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigt z.B. die folgende Verbindung der Herstellungsbeispiele überlegene Wirkung gegenüber dem Stand der Technik: 1.

Beispiel L

Test mit Lucilia cuprina Larven (OP-res. Goondiwindi-Stamm)

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1, 3.

0243636

**Beispiel M**

Test mit Stomoxys calcitrans

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z.B. die folgenden Verbindungen der Herstellungsbeispiele überlegene Wirkung gegenüber dem Stand der Technik: 1

0243636

Beispiel N

Facefly-Test (Musca autumnalis)

| | | |
|---|---|---|
| Emulgator: | 35 Gewichtsteile | Ethylenglykolmonomethylether |
| | 35 Gewichtsteile | Nonylphenolpolyglykolether |

Zur Herstellung einer geeigneten Formulierung vermischt man drei Gewichtsteile Wirkstoff mit sieben Gewichtsteilen des oben angegebenen Lösungsmittel-Emulgator-Gemisches und verdünnt das so erhaltene Emulsionskonzentrat mit Wasser auf die jeweils gewünschte Konzentration.

10 adulte Faceflies (Musca autumnalis) werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad in Prozent bestimmt, wobei 100 % bedeuten, daß alle und 0 %, daß keine Fliegen abgetötet worden sind.

Bei diesem Test zeigt z.B. die folgende Verbindung der Herstellungsbeispiele überlegene Wirkung gegenüber dem Stand der Technik: 1.

Beispiel O

Test mit Psoroptes ovis

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat auf die gewünschte Konzentration.

Etwa 10 - 25 Psoroptes ovis in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 3.

Patentansprüche

1. Insektizide und akarizide Mittel, gekennzeichnet durch einen Gehalt an mindestens einem 5-Amino-1-aryl-pyrazol der allgemeinen Formel

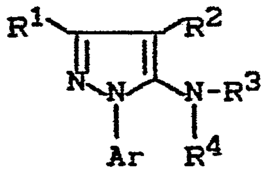

(I)

in welcher

$R^1$ für Wasserstoff, Alkyl oder Halogenalkyl steht,

$R^2$ für Trifluormethyl steht,

$R^3$ für Wasserstoff, Alkyl oder Alkenyl steht,

$R^4$ für Wasserstoff, Alkyl, Alkenyl, Alkinyl oder einen Rest 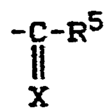 steht,

Ar für jeweils gegebenenfalls substituiertes Phenyl oder Pyridyl steht,

wobei X für Sauerstoff oder Schwefel steht,

und $R^5$ Alkyl oder Halogenalkyl bedeutet.

2. Insektizide und akarizide Mittel gemäß Anspruch 1, dadurch gekennzeichnet, daß in der Formel (I)

$R^1$ für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen oder für Wasserstoff steht,

$R^2$ für Trifluormethyl steht,

$R^3$ für Wasserstoff, $C_1$-$C_6$-Alkyl oder $C_2$-$C_6$-Alkenyl steht,

$R^4$ für Wasserstoff, $C_1$-$C_6$-Alkyl, $C_2$-$C_6$-Alkenyl, $C_2$-$C_6$-Alkinyl oder einen Rest $-C(=O)-R^5$ steht,

wobei $R^5$ für $C_1$-$C_4$-Alkyl oder $C_1$-$C_4$-Halogenalkyl steht,

Ar für jeweils gegebenenfalls einfach oder mehrfach, gleich oder verschieden substituiertes Phenyl, 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen, außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_m-R^6$

wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht.

3. Insektizide und akarizide Mittel gemäß Anspruch 1 oder 2, dadurch gekennzeichnet, daß in der Formel (I)

$R^1$ für Wasserstoff, Methyl, Ethyl, n- oder i-Propyl oder Trifluormethyl steht,

$R^2$ für Trifluormethyl steht,

$R^3$ für Wasserstoff, Methyl, Ethyl, n- oder i-Propyl, n-, i-, s- oder t-Butyl, n- oder i-Pentyl, n- oder i-Hexyl, Vinyl, Propenyl-1, Propenyl-2, Butenyl-1, Butenyl-2, steht,

$R^4$ für Wasserstoff, Methyl, Ethyl, n- oder i-Propyl, n-, i-, s- oder t-Butyl, n- oder i-Pentyl, n- oder i-Hexyl, Propargyl, Butinyl-1, Butinyl-2 oder für einen der folgenden Reste steht:

$-\underset{\underset{O}{\|}}{C}-CH_3$ , $-\underset{\underset{O}{\|}}{C}-C_2H_5$ , $-\underset{\underset{O}{\|}}{C}-C_3H_7\text{-n}$ , $-\underset{\underset{O}{\|}}{C}-C_3H_7\text{-i}$ ,

$-\underset{\underset{O}{\|}}{C}-C_4H_9\text{-n}$ , $-\underset{\underset{O}{\|}}{C}-C_4H_9\text{-s}$ , $-\underset{\underset{O}{\|}}{C}-C_4H_9\text{-t}$ , $-\underset{\underset{O}{\|}}{C}-CF_3$

Ar für jeweils gegebenenfalls ein- bis fünffach, gleich oder verschieden substituiertes Phenyl, 2-Pyridyl, 3-Pyridyl oder 4-Pyridyl steht, wobei als Substituenten in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^6$, wobei

$R^6$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht

und

m für eine Zahl 0, 1 oder 2 steht.

4. Verfahren zur Bekämpfung von Insekten und/oder Spinnentieren dadurch gekennzeichnet, daß man 5-Amino-1-aryl-pyrazole der Formel (I) auf Insekten und/oder Spinnentieren und/oder deren jeweiligen Lebensraum einwirken läßt.

5. Verwendung von 5-Amino-1-aryl-pyrazolen der Formel (I) zur Bekämpfung von Insekten und/oder Spinnentieren.

6. Verfahren zur Herstellung von insektiziden und/oder akariziden Mitteln, dadurch gekennzeichnet, daß man 5-Amino-1-phenyl-pyrazole gemäß Formel (I) mit Streckmitteln und/oder oberflächenaktiven Mitteln vermischt.

7. 5-Amino-1-phenyl-pyrazole der Formel

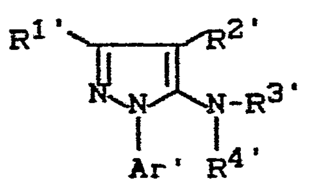

(Ia)

in welcher

$R^{1'}$ für Wasserstoff, Alkyl oder Halogenalkyl steht,

$R^{2'}$ für Trifluormethyl steht,

$R^{3'}$ für Wasserstoff, Alkyl oder Alkenyl steht,

$R^{4'}$ für Wasserstoff, Alkyl, Alkenyl, Alkinyl oder einen Rest

$$-\underset{\underset{X}{\|}}{C}-R^{5'}$$ steht

Ar' für jeweils gegebenenfalls substituiertes Phenyl oder Pyridyl steht,

wobei

X für Sauerstoff oder Schwefel steht und

$R^{5'}$ für Alkyl oder Halogenalkyl steht,

wobei, falls $R^{1'}$ für Wasserstoff steht, die Reste $R^{3'}$, $R^{4'}$ und Ar' nicht die folgenden Bedeutungskombinationen besitzen dürfen:

$R^{3'}$ = Wasserstoff, $R^{4'}$ = Wasserstoff und Ar' = 2,4,6-Trichlorphenyl oder 2,4,6-Trinitrophenyl;

$R^{3'}$ = Wasserstoff, $R^{4'}$ = $COCH_3$ und Ar' = 2,6-Dichlor-4-trifluormethylphenyl oder 2,6-Dichlor-4-trifluormethyloxyphenyl;

$R^{3'}$ = Wasserstoff, $R^{4'}$ = $COC_2H_5$ und Ar' = 2,6-Dichlor-4-trifluormethylthiophenyl oder 2,3,6-Trifluor-4-trifluormethylthiophenyl;

$R^{3'}$ = Methyl, $R^{4'}$ = $COCH_3$ und Ar' = 2-Chlor-4-trifluormethylphenyl.

8. Verfahren zur Herstellung von 5-Amino-1-phenyl-pyrazolen der Formel

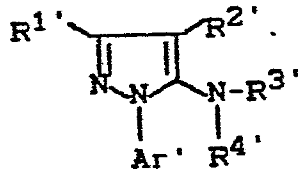

(Ia)

in welcher

$R^{1'}$ für Wasserstoff, Alkyl oder Halogenalkyl steht,

$R^{2'}$ für Trifluormethyl steht,

$R^{3'}$ für Wasserstoff, Alkyl oder Alkenyl steht,

$R^{4'}$ für Wasserstoff, Alkyl, Alkenyl, Alkinyl oder einen Rest

$$-\underset{\underset{X}{\|}}{C}-R^{5'}$$ steht

Ar' für jeweils gegebenenfalls substituiertes Phenyl oder Pyridyl steht,

wobei

X für Sauerstoff oder Schwefel steht und

$R^{5'}$ für Alkyl oder Halogenalkyl steht,

wobei, falls $R^{1'}$ für Wasserstoff steht, die Reste $R^{3'}$, $R^{4'}$ und Ar' nicht die folgenden Bedeutungskombinationen besitzen dürfen:

$R^{3'}$ = Wasserstoff, $R^{4'}$ = Wasserstoff und Ar' = 2,4,6-Trichlorphenyl oder 2,4,6-Trinitrophenyl;

$R^{3'}$ = Wasserstoff, $R^{4'}$ = $COCH_3$ und Ar' = 2,6-Dichlor-4-trifluormethylphenyl oder 2,6-Dichlor-4-trifluormethyloxyphenyl;

$R^{3'}$ = Wasserstoff, $R^{4'}$ = $COC_2H_5$ und Ar' = 2,6-Dichlor-4-trifluormethylthiophenyl oder 2,3,6-Trifluor-4-trifluormethylthiophenyl;

$R^{3'}$ = Methyl, $R^{4'}$ = $COCH_3$ und Ar' = 2-Chlor-4-trifluormethylphenyl,

dadurch gekennzeichnet, daß man

(a) zum Erhalt der 1-Aryl-5-amino-4-trifluormethyl-pyrazole der Formel (Ia), 1-Aryl-5-amino-4-carboxypyrazole der Formel (II)

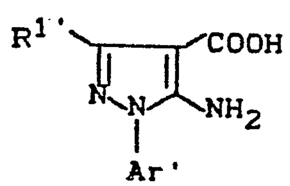

(II)

in welcher

$R^{1'}$ und Ar' die oben angegebene Bedeutung besitzen,

mit Schwefeltetrafluorid in Fluorwasserstoffsäure unter erhöhtem Druck umsetzt,

und die entstehenden Verbindungen der Formel

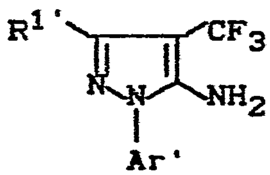

(Ib)

gegebenenfalls alkyliert, alkenyliert, alkinyliert oder acyliert,

oder daß man

(b) zum Erhalt von 1-Aryl-5-amino-4-trifluormethyl-pyrazolen der Formel

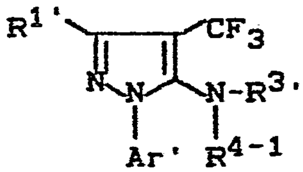

(Ic)

in welcher

$R^{4-1}$ für Alkyl, Alkenyl, Alkinyl oder für einen Rest $-\underset{\underset{X}{\|}}{C}-R^{5'}$ steht und

$R^{1'}$, $R^{3'}$, $R^{5'}$, Ar' und X die oben angegebene Bedeutung haben,

1-Aryl-5-amino-4-trifluormethylpyrazole der Formel

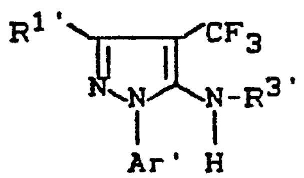

(Id)

in welcher

$R^{1'}$, $R^{3'}$ und Ar' die oben angegebene Bedeutung haben,

mit Verbindungen der Formel

$$R^{4-1}\text{-}A \qquad \text{(III)}$$

in welcher

$R^{4-1}$ für Alkyl, Alkenyl, Alkinyl oder für einen Rest $-\underset{\underset{X}{\|}}{C}-R^{5'}$ steht, wobei

$R^{5'}$ und X die oben angegebene Bedeutung haben und

A für eine elektronenziehende Abgangsgruppe steht,

gegebenenfalls in Gegenwart eines Verdünnungsmittels und gegebenenfalls in Gegenwart eines Säurebindemittels umsetzt,

oder daß man

(c) zum Erhalt der 1-Aryl-5-amino-4-trifluormethyl-pyrazole der Formel

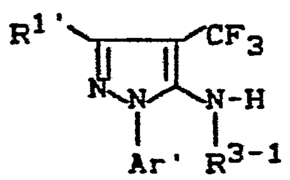

(Ie)

in welcher

$R^{1'}$ und Ar' die oben angegebene Bedeutung haben und

$R^{3-1}$ für Alkyl oder Alkenyl steht,

5-(N-Acylamino)-4-trifluormethyl-1-aryl-pyrazole der Formel

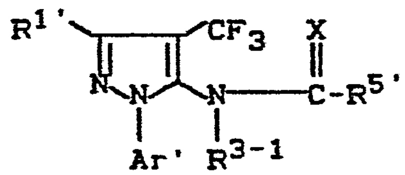

(If)

in welcher

$R^{1'}$, $R^{5'}$, $R^{3-1}$, X und Ar' die oben angegebene Bedeutung haben,

mit Säuren oder Basen, gegebenenfalls in Gegenwart eines Verdünnungsmittels deacyliert,

oder daß man

(d) zum Erhalt der 1-Aryl-5-amino-trifluormethyl-pyrazole der Formel

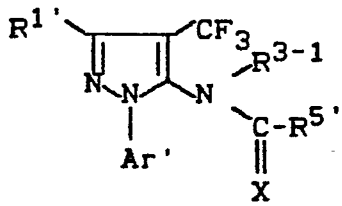

(Ig)

in welcher

$R^{1'}$, $R^{3-1}$, $R^{5'}$, X und Ar' die oben angegebene Bedeutung haben,

1-Aryl-5-amino-trifluormethylpyrazole der Formel

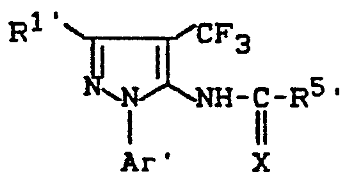

(Ih)

in welcher

$R^{1'}$, $R^{5'}$, X und Ar' die oben angegebene Bedeutung besitzen,

mit Verbindungen der Formel

$$R^{3-1}-A$$

in welcher

$R^{3-1}$ die oben angegebene Bedeutung besitzt und A für eine Abgangsgruppe steht, umsetzt.

Europäisches Patentamt

**0243636**

# EUROPÄISCHER RECHERCHENBERICHT

Nummer der Anmeldung

EP 87 10 3585

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 154 115 (BAYER)<br>* Seite 13 *<br>----- | 7 | C 07 D 231/40<br>C 07 D 231/38<br>C 07 D 401/04<br>A 01 N 43/56<br>A 01 N 43/40 |
| | | | **RECHERCHIERTE SACHGEBIETE (Int. Cl.4)** |
| | | | C 07 D 231/00<br>C 07 D 401/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 28-06-1987 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82